# FASCICULE DE BREVET EUROPEEN

(11) **EP 0 984 731 B1**
(45) Date de publication et mention de la délivrance du brevet: **21.07.2004**
(21) Numéro de dépôt: 98925727.4
(22) Date de dépôt: 15.05.1998
(51) Int. Cl.: A61B 17/70

(54) **IMPLANT POUR DISPOSITIF D'OSTEOSYNTHESE, DU TYPE A CROCHET**
HAKEN FÜR DIE OSTEOSYNTHESE DER WIRBELSÄULE
IMPLANT FOR OSTEOSYNTHESIS DEVICE WITH HOOK

(30) Priorité: 16.05.1997 FR 9706027
(43) Date de publication de la demande: 15.03.2000
(73) Titulaire: SCIENT'X, 75007 Paris (FR)
(72) Inventeur: ALBY, Albert, F-75116 Paris (FR)
(74) Mandataire: Thibault, Jean-Marc
(86) Numéro de dépôt international: PCT/FR1998/000979
(87) Numéro de publication internationale: WO 1998/052483

(56) Documents cités:
- WO-A-91/01691
- FR-A- 2 695 550
- US-A- 5 454 812

## Description

### DOMAINE TECHNIQUE :

La présente invention concerne un implant pour dispositif d'ostéosynthèse, notamment du rachis, comprenant un dispositif d'ancrage osseux surmonté d'une tête de fixation constituée par deux branches latérales formant un U ouvert et destiné à recevoir une tige de liaison en vue de son immobilisation par serrage, par l'intermédiaire d'un écrou fileté, apte à se visser sur des parties filetées correspondantes réalisées sur les parois des branches latérales de la tête de fixation.

### TECHNIQUE ANTERIEURE :

Il est connu d'utiliser, en général, comme dispositif d'ancrage, des vis pédiculaires ou des crochets. De nombreux perfectionnements concernent les vis pédiculaires qui présentent l'avantage d'assurer une fixation effective de l'implant puisque la vis pédiculaire est ancrée par vissage dans un pédicule.

Néanmoins, il n'est pas toujours possible d'utiliser des vis pédiculaires, notamment lorsqu'il s'agit de vertèbre d'extrémité du rachis, ou en raison de la réglementation de certains pays qui autorise de manière très limitée, l'utilisation de vis pédiculaires, leur emploi étant considéré comme délicat et donc dangereux pour le patient.

Il est donc dans certains cas, nécessaire d'avoir recours à des crochets.

Les crochets utilisés habituellement sont conformés pour s'accrocher sur une partie saillante des os de la vertèbre tel que le pied du pédicule ou pour se fixer sur l'arche vertébrale, soit du côté infralamaire, soit du côté supralamaire.

Néanmoins, force est de constater que l'utilisation des crochets présente bon nombre d'inconvénients car un crochet isolé ne saurait à lui seul réaliser une fixation mécanique effective comme peut le faire une vis. En fait, il ne peut agir efficacement qu'en tant que butée utilisée en association avec un autre crochet, en vue d'obtenir un effet serre-joint devenant aussi efficace qu'une vis.

Un crochet comprend un corps de crochet comportant une face intérieure d'appui et une face extérieure de fixation munie de moyens de fixation à une tige de solidarisation de points d'ancrage. C'est la tige les reliant entre-eux qui assure leur maintien sur deux vertèbres successives.

Un tel montage a pour effet de solidariser, en les rapprochant l'une vers l'autre, deux vertèbres successives, sans possibilité d'agir séparément sur chacune d'elles. De plus, il faut trouver un appui correct des deux crochets simultanément et la stabilisation latérale est insuffisante.

Il a déjà été proposé des dispositifs d'ancrage par crochets comportant des dispositifs pour améliorer leur stabilité. Ainsi, la demande de brevet internationale **WO-A-91/01 691** décrit un dispositif comportant deux crochets en vis-à-vis situés de part et d'autre de la vertèbre, reliés entre-eux par une vis de serrage destinée à les rapprocher à la demande, chaque crochet comportant une tête de fixation dans laquelle est engagée une tige de liaison et l'immobilisation de l'ensemble s'effectuant par serrage des écrous de chacun desdits crochets.

Un tel mode de réalisation présente l'inconvénient majeur de devoir intervenir en serrage sur deux crochets et de rester tributaire de la tige de liaison pour obtenir un serrage effectif, ce qui revient à dire qu'avant cette opération, le dispositif demeure instable.

On retrouve ce système de pince dans les documents **FR 2 695 550** ou **FR 2 718 944**.

Il est également connu, par exemple par le brevet **FR 2 695 550,** un implant autonome, ayant des moyens de fixation effectifs qui lui sont propres et qui s'affranchissent de la tige de liaison destinée à relier deux ou plusieurs vertèbres successives. Ce brevet décrit une pince pédiculaire comprenant un crochet solidaire d'une tête de fixation constituée par deux branches latérales en U ouvert et destinée à recevoir une tige de liaison en vue de son immobilisation, par l'intermédiaire d'un écrou fileté apte à se visser sur des parties filetées correspondantes, réalisées sur les branches latérales de la tête de fixation. Cet implant comporte également un contre-crochet dirigé vers le crochet et connecté avec ce dernier par l'intermédiaire d'une tige filetée. Le crochet et le contre-crochet sont destinés à enserrer une vertèbre à la manière d'un serre-joint agissant de part et d'autre de ladite vertèbre, indépendamment de la tige de liaison.

Cet implant présente un inconvénient majeur relatif à la possibilité de déplacement relatif entre le crochet et le contre-crochet. Il apparaît également que l'opération de réglage de l'écartement entre le crochet et le contre-crochet pour ce type d'implant ne constitue pas une opération simple à mener à bien.

Dans le même sens, le brevet US 5 454 812 décrit un dispositif de liaison intervertébrale comportant un crochet fixe sur lequel est monté un lien formant boucle sur laquelle est monté un crochet d'ajustement et un élément de blocage en position du crochet mobile. La pièce de blocage est destinée à venir en butée contre le crochet mobile après son positionnement sur les vertèbres et à subir une déformation pour permettre sa fixation sur le lien.

### EXPOSE DE L'INVENTION :

L'objet de l'invention vise donc à remédier aux inconvénients énoncés ci-dessus en proposant un implant pour dispositif d'ostéosynthèse mettant en oeuvre un crochet et un contre-crochet, indépendants de la tige de liaison et offrant l'avantage de permettre, d'une part, un réglage aisé de l'écartement entre le crochet et le contre-crochet, ainsi que leur maintien en position relative et, d'autre part, une immobilisation sûre et efficace entre le crochet et le contre-crochet.

L'objet de l'invention propose ainsi une solution pour assurer, d'une part, la stabilisation primaire de l'ancrage osseux par crochets en améliorant l'ancrage osseux dès l'étape préliminaire de pose de l'implant sur le rachis, pendant l'étape de réduction de déformation du rachis et pendant toute la durée ultérieure du maintien du rachis et, d'autre part, une stabilité fiable et permanente de l'ancrage pour éviter les pertes de réduction à court ou moyen terme.

Pour atteindre cet objet, l'implant comprend :
- un crochet solidaire d'une tête de fixation constituée par deux branches latérales en U ouvert et destinée à recevoir une tige de liaison en vue de son immobilisation, par l'intermédiaire d'un écrou fileté apte à se visser sur des parties filetées correspondantes réalisées sur les branches latérales de la tête de fixation,
- un contre-crochet dirigé vers le crochet et connecté librement avec ledit crochet pour permettre au crochet et au contre-crochet d'enserrer au moins une vertèbre à la manière d'un serre-joint, indépendamment de la tige de liaison,
- et des moyens de blocage du crochet par rapport au contre-crochet, en vue d'assurer leur immobilisation relative.

Selon l'invention, l'implant comporte :
- des moyens de connexion libre entre le crochet et le contre-crochet, constitués par l'intermédiaire d'un doigt prolongeant l'une des extrémités du contre-crochet et adapté pour coulisser dans un alésage réalisé dans une partie supérieure du crochet avec la tête de fixation selon un axe Y-Y' perpendiculaire à l'axe commun X-X' de la tête de fixation et du crochet,
- des moyens de maintien en position relative du crochet et du contre-crochet.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.

### BREVE DESCRIPTION DES DESSINS :

La **fig. 1** est une vue en plan d'un mode de réalisation de l'invention.
La **fig. 2** est une vue en coupe de la partie supérieure de l'implant selon la ligne brisée II-II de la **fig. 1**.
La **fig. 3** est une vue en plan d'un autre mode de réalisation de l'invention sur laquelle ne figurent ni l'écrou, ni la tige de liaison.
La **fig. 4** est une vue en plan selon un autre mode de réalisation de l'invention sur laquelle ne figurent ni l'écrou, ni la tige de liaison.
La **fig. 5** est une vue de dessus du mode de réalisation de la **fig. 4**.

### MEILLEURE MANIERE DE REALISER L'INVENTION :

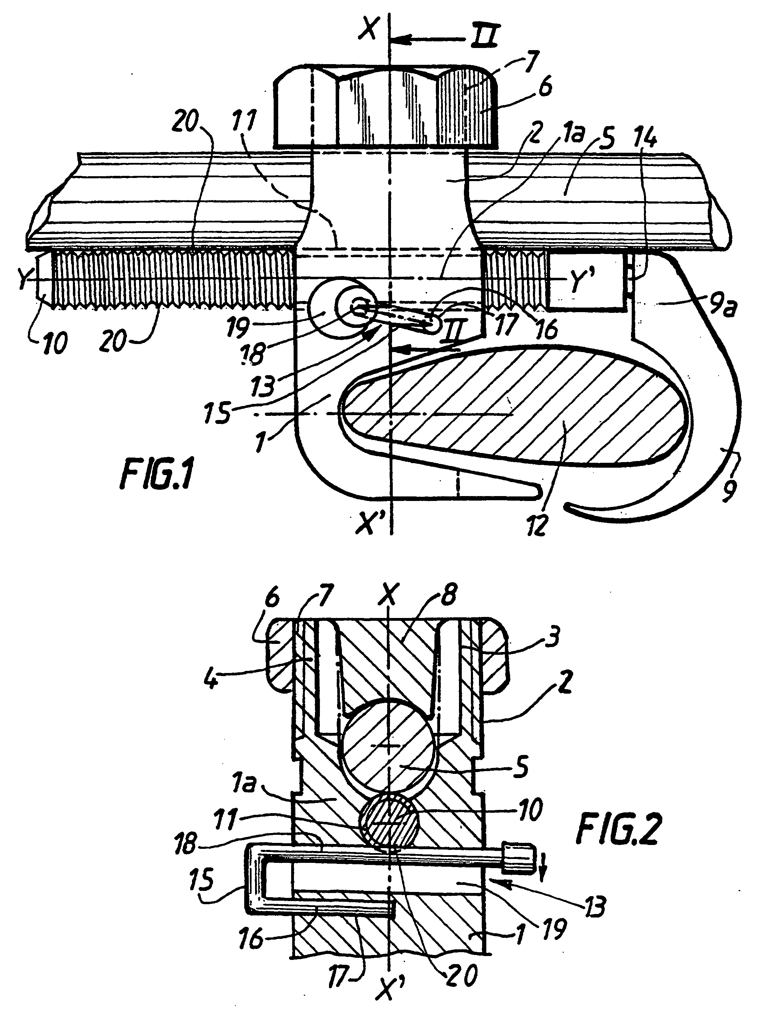

L'implant représenté sur les **fig. 1** et **2** comprend un crochet de référence **1** et un contre-crochet **9**. Le crochet **1** est solidaire d'une tête de fixation **2**, qui est constituée, de manière connue, par deux branches latérales **3**, **4** en forme de U ouvert et destinées à recevoir entre elles une tige de liaison **5**, en vue de son immobilisation, par l'intermédiaire d'un écrou fileté **6** apte à se visser sur des parties filetées correspondantes **7** réalisées dans l'exemple illustré, sur les parois extérieures partiellement cylindriques des branches latérales **3, 4** de la tête de fixation **2.**

Selon l'exemple de réalisation représenté sur les **fig. 1** et **2,** l'écrou **6** comporte dans sa zone diamétrale, un patin **8** monté libre en rotation et destiné à coopérer en serrage avec la tige de liaison **5.**

L'implant comprend des moyens de connexion libre entre le crochet **1** et le contre-crochet **9** permettant leur libre rapprochement ou écartement. Le contre-crochet **9** est dirigé vers le crochet **1** et coopère librement avec lui par l'intermédiaire d'un doigt **10.** Le doigt **10** prolonge l'une des extrémités **9a** du contre-crochet **9** et coulisse dans un alésage, un trou cylindrique ou un perçage cylindrique **11** réalisé dans la partie supérieure **1a** du crochet **1** et situé sensiblement dans le plan du crochet **1.**

Ainsi, le coulissement du doigt **10** du contre-crochet **9** dans l'alésage **11** du crochet **1,** permet au crochet **1** et au contre-crochet **9** d'enserrer au moins une vertèbre **12** à la manière d'un serre-joint agissant de part et d'autre de la vertèbre **12,** indépendamment de la tige de liaison **5.** Le maintien en place de l'ensemble crochet **1**/contre-crochet **9** sur la vertèbre **12** est obtenu par des moyens **13** qui leur sont propres. L'implant selon l'invention comporte ainsi des moyens **13** adaptés pour assurer un maintien en position relative, du crochet **1** par rapport au contre-crochet **9.**

Selon ce même exemple de réalisation représenté sur les **fig. 1** et **2,** l'axe Y-Y' de l'alésage **11** du crochet **1** avec lequel coopère le doigt **10** du contre-crochet **9,** est perpendiculaire à l'axe vertical commun **X**-**X'** de la tête de fixation **2** et du crochet **1,** et se situe dans un plan identique à celui du crochet **1** mettant ainsi crochet **1** et contre-crochet **9** en vis-à-vis.

Selon une variante de réalisation non représentée, l'axe **Y**-**Y'** de l'alésage **11** demeure perpendiculaire à l'axe vertical **X**-**X'** de la tête de fixation **2** et du crochet **1**, mais se situe dans un plan différent de celui du crochet **1,** mettant ainsi le crochet **1** et le contre-crochet **9** en décalage angulaire l'un par rapport à l'autre et selon un sens dit "à droite" ou un sens dit "à gauche".

Selon une autre variante de l'invention, le contre-crochet **9** est relié au doigt **10** par l'intermédiaire d'une articulation **14**, le rendant libre en rotation par rapport à l'axe **Y-Y'.**

Selon une autre variante de l'invention, la tête de fixation **2** est montée libre en rotation par rapport au crochet **1.**

Les moyens **13** de maintien en place du crochet **1** et du contre-crochet **9** sont formés par un élément déformable élastiquement constitué, dans l'exemple illustré aux **fig. 1** et **2,** par une épingle **15.**

Cette épingle comprend une branche **16** fixée dans un logement **17** dont l'axe longitudinal s'étend de manière sensiblement perpendiculaire au plan du crochet **1** dans la partie supérieure **1a.** L'autre branche **18** de l'épingle **15** est déformable élastiquement et se loge librement dans un trou cylindrique **19,** sensiblement parallèle au premier logement **17** et percé dans la partie supérieure **1a** du crochet **1.** Les trous **11** et **19** sont sécants et sensiblement perpendiculaires. De cette manière, la branche élastique **18** peut coopérer à la manière d'un cliquet, avec des crans **20** ménagés, par exemple, transversalement sur le doigt **10** du contre-crochet **9** lors du serrage, par déplacement vers le crochet **1** ou permet la libération du doigt lors de son abaissement. Il doit être compris que l'épingle **15** autorise un rapprochement relatif du crochet **1** et du contre-crochet **9**, mais interdit leur écartement relatif si aucune action n'est exercée sur l'élément déformable élastiquement qui agit comme un système anti-retour.

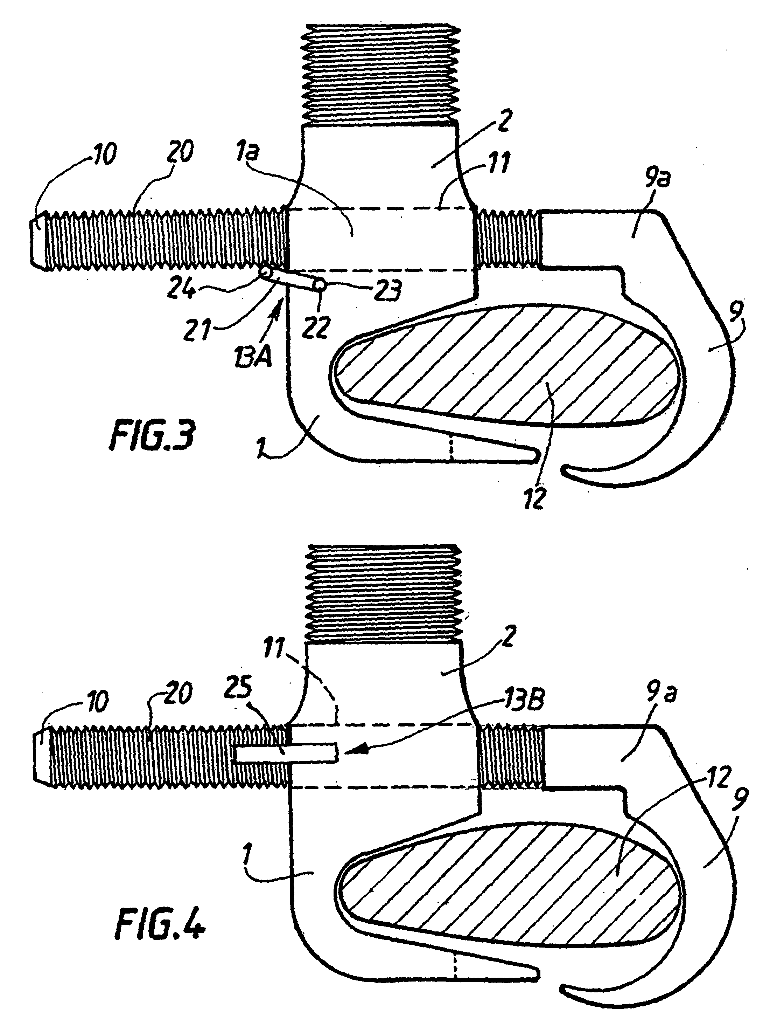

Selon le mode de réalisation représenté sur la **fig. 3,** les moyens de maintien en place **(13A)** du crochet **1** et du contre-crochet **9** sur une vertèbre donnée **12,** sont constitués par un élément déformable élastiquement formé par une épingle **21,** dont une branche **22** est fixée dans un logement **23** dont l'axe s'étend de manière sensiblement perpendiculaire au plan du crochet **1** dans la partie supérieure **1a ;** l'autre branche **24** est déformable élastiquement et s'étend librement et parallèlement à la première à l'extérieur du crochet **1** pour venir coopérer avec le doigt **10** du contre-crochet **9** qui lui est sensiblement perpendiculaire. De cette manière, la branche élastique **24** peut coopérer à la manière d'un cliquet, avec les crans **20** ménagés transversalement sur le doigt **10** du contre-crochet **9** lors du serrage, par déplacement vers le crochet **1** ou permet la libération du doigt lors de son abaissement.

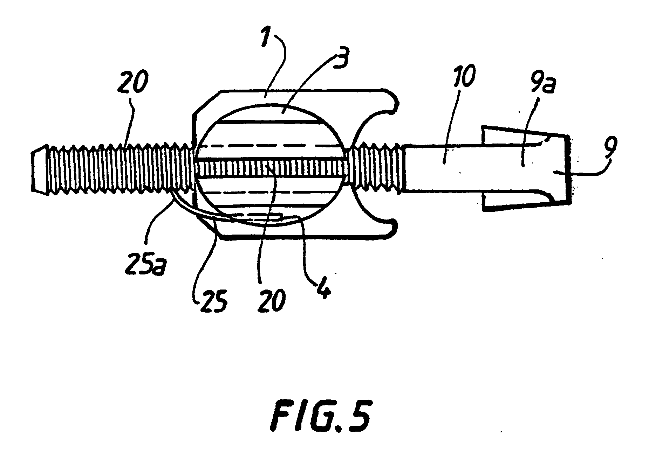

Selon le mode de réalisation représenté sur les **fig. 4** et **5,** les moyens de maintien en place **(13B)** du crochet **1** et du contre-crochet **9** sur une vertèbre donnée **12,** sont constitués par une lame déformable élastiquement **25** fixée de manière parallèle au plan du crochet **1,** parallèlement au doigt **10** du contre-crochet **9** et dont l'extrémité libre **25a** est recourbée en direction du doigt **10.** De cette manière, l'extrémité libre **25a** peut coopérer à la manière d'un cliquet, avec les crans **20** ménagés transversalement sur le doigt **10** du contre-crochet **9** lors du serrage, par déplacement vers le crochet **1** ou permet la libération du doigt par traction sur la lame **25**.

Selon une caractéristique commune à tous les modes de réalisation précités et visibles particulièrement sur la **fig. 2**, l'implant comporte des moyens de blocage en position définitive du crochet **1** et du contre-crochet **9**, en vue d'assurer leur immobilisation relative. Selon une caractéristique préférée de réalisation, le serrage de la tige de liaison **5** entraîne simultanément le blocage par cette dernière, du doigt **10**. L'alésage **11** est réalisé de manière sécante avec le fond du **U** défini par les branches latérales **3**, **4** de la tête de fixation **2**, destinée à recevoir la tige de liaison **5**, de manière à ce que le serrage de ladite tige **5** par l'écrou **6**, assure simultanément le serrage définitif de ladite tige **5** et du doigt **10** sur le crochet **1**. Le crochet **1** et le contre-crochet **9** sont ainsi immobilisés définitivement après avoir été préalablement mis en place et maintenus en position à l'aide de l'élément déformable élastiquement.

Le contre-crochet du type précité pourrait être associé à une vis pédiculaire traditionnelle de manière à renforcer la fixation de celle-ci sur une vertèbre donnée. De plus, il est à noter que le crochet **1** et le contre-crochet **9** peuvent être destinés à enserrer une seule vertèbre ou plusieurs vertèbres.

L'implant selon l'invention permet ainsi de régler facilement l'écartement entre le crochet **1** et le contre-crochet **9** par un simple déplacement relatif entre le crochet et le contre-crochet destinés à enserrer une ou plusieurs vertèbres. Le crochet **1** et le contre-crochet **9** sont maintenus dans cette position stable pendant toute la durée de l'étape préliminaire de pose de l'implant et de la réduction de la déformation du rachis. L'immobilisation définitive et fiable du crochet et du contre-crochet est réalisée au cours de l'étape ultérieure de fixation définitive de l'implant.

## Revendications

1. Implant pour dispositif d'ostéosynthèse, notamment du rachis, comprenant :
- un crochet **(1)**, solidaire d'une tête de fixation **(2)** constituée par deux branches latérales **(3, 4)** en U ouvert et destinée à recevoir une tige de liaison **(5)** en vue de son immobilisation, par l'intermédiaire d'un écrou fileté **(6)** apte à se visser sur des parties filetées correspondantes **(7)** réalisées sur les branches latérales **(3**, **4)** de la tête de fixation,
- un contre-crochet **(9)** dirigé vers le crochet **(1)** et connecté librement avec ledit crochet **(1)** pour permettre au crochet **(1)** et au contre-crochet **(9)** d'enserrer au moins une vertèbre **(12)** à la manière d'un serre-joint, indépendamment de la tige de liaison (**5**),
- et des moyens de blocage du crochet **(1)** par rapport au contre-crochet **(9)**, en vue d'assurer leur immobilisation relative,
**caractérisé en ce qu'**il comporte :
- des moyens de connexion libre entre le crochet **(1)** et le contre-crochet **(9)**, constitués par l'intermédiaire d'un doigt **(10)** prolongeant l'une des extrémités **(9a)** du contre-crochet et adapté pour coulisser dans un alésage **(11)** réalisé dans une partie supérieure **(1a)** du crochet **(1)** avec la tête de fixation **(2)** selon un axe Y-Y' perpendiculaire à l'axe commun X-X' de la tête de fixation **(2)** et du crochet **(1)**,
- des moyens **(13, 13A, 13B)** de maintien en position relative du crochet **(1)** et du contre-crochet **(9)**.

2. Implant selon la revendication 1, **caractérisé en ce que** les moyens **(13, 13A, 13B**) de maintien en position sont constitués par un élément déformable élastiquement **(15, 21 25)** porté par le crochet et destiné, d'une part, à coopérer à la manière d'un cliquet, avec des crans **(20)** ménagés sur le doigt **(10),** lors du serrage, par rapprochement du crochet et du contre-crochet et, d'autre part, à permettre la libération du doigt **(10)** par une action sur l'élément déformable.

3. Implant selon la revendication 1, **caractérisé en ce que** les moyens de blocage en position définitive du crochet **(1)** et du contre-crochet **(9)** sont constitués par l'intermédiaire de la tige de liaison **(5)** dont le serrage assure le blocage du doigt **(10).**

4. Implant selon la revendication 2 ou 3, **caractérisé en ce que** le doigt **(10)** est adapté pour coulisser à l'intérieur de l'alésage **(11)** aménagé de manière sécante avec le fond du U défini par les branches latérales **(3, 4)** et destiné à recevoir la tige de liaison **(5),** de manière que le serrage de ladite tige **(5)** par l'écrou **(6),** assure simultanément le serrage définitif de la tige de liaison **(5)** et du doigt **(10)** sur le crochet **(1).**

5. Implant selon la revendication 1, **caractérisé en ce que** l'axe **(Y-Y')** de l'alésage **(11)** du crochet **(1)** avec lequel coopère le doigt **(10)** du contre-crochet **(9),** est perpendiculaire à l'axe vertical commun **(X-X')** de la tête de fixation **(2)** et du crochet **(1),** et se situe dans un plan identique à celui du crochet **(1)** mettant ainsi le crochet **(1)** et contre-crochet **(9)** en vis-à-vis.

6. Implant selon la revendication 1, **caractérisé en ce que** l'axe **(Y-Y')** de l'alésage **(11)** du crochet **(1)** avec lequel coopère le doigt **(10)** du contre-crochet **(9),** est perpendiculaire à l'axe vertical commun **(X-X')** de la tête de fixation **(2)** et du crochet **(1),** et se situe dans un plan différent du plan de symétrie du crochet **(1)** mettant ainsi les crochet **(1)** et contre-crochet **(9)** en décalage angulaire l'un par rapport à l'autre et selon un sens dit "à droite" ou un sens dit "à gauche".

7. Implant selon l'une des revendications 1 à 5, **caractérisé en ce que** le contre-crochet **(9)** est relié au doigt **(10)** par l'intermédiaire d'une articulation **(14),** le rendant libre en rotation par rapport à l'axe **(Y-Y').**

8. Implant selon l'une des revendications 1 à 6, **caractérisé en ce que** la tête de fixation **(2)** est montée libre en rotation par rapport au crochet **(1).**

9. Implant selon l'une des revendications 2 à 8, **caractérisé en ce que** l'élément déformable élastiquement est constitué par une épingle **(15),** dont une branche **(16)** est fixée dans un logement **(17)** dont l'axe longitudinal s'étend de manière sensiblement perpendiculaire au plan du crochet **(1)** dans la partie supérieure **(1a)** dudit crochet **(1),** et dont l'autre branche **(18)** est déformable élastiquement et se loge librement dans un trou cylindrique **(19),** sensiblement parallèle au premier logement **(17),** percé dans la partie supérieure **(1a)** du crochet **(1),** l'alésage **(11)** et le trou cylindrique **(19)** étant sécants et sensiblement perpendiculaires, afin que la branche élastique **(18)** puisse coopérer à la manière d'un cliquet, avec des crans **(20)** ménagés transversalement sur le doigt **(10)** du contre-crochet **(9)** lors du serrage, par déplacement vers le crochet (**1**) ou permettre la libération du doigt **(10)** par abaissement de ladite branche élastique **(18).**

10. Implant selon l'une des revendications 2 à 8, **caractérisé en ce que** l'élément déformable élastiquement est constitué par une épingle **(21),** dont une branche **(22)** est fixée dans un logement **(23)** dont l'axe s'étend de manière sensiblement perpendiculaire au plan du crochet **(1)** dans la partie supérieure **(1a)** et dont l'autre branche **(24)** est déformable élastiquement et s'étend librement et parallèlement à la première à l'extérieur du crochet **(1)** pour venir coopérer avec le doigt **(10)** du contre-crochet **(9)** qui lui est sensiblement perpendiculaire, afin que ladite branche élastique **(24)** puisse coopérer, à la manière d'un cliquet, avec des crans **(20)** ménagés transversalement sur le doigt **(10)** du contre-crochet **(9)** lors du serrage par déplacement vers le crochet **(1)** ou permettre la libération du doigt **(10)** par abaissement de ladite branche élastique **(24).**

11. Implant selon l'une des revendications 2 à 8, **caractérisé en ce que** l'élément déformable élastiquement est constitué par une lame déformable élastiquement **(25)** fixée de manière parallèle au plan du crochet **(1)**, parallèlement au doigt **(10)** du contre-crochet **(9)** et dont l'extrémité libre **(25a)** est recourbée en direction du doigt **(10)**, afin que ladite extrémité libre **(25a)** puisse coopérer, à la manière d'un cliquet, avec des crans **(20)** ménagés transversalement sur le doigt **(10)** du contre-crochet **(9)** lors du serrage par déplacement vers le crochet **(1)** ou permettre la libération du doigt par traction sur la lame **(25).**

12. Implant selon l'une des revendications 1 à 11, **caractérisé en ce que** le contre-crochet **(9)** est équipé d'une vis pédiculaire.

## Patentansprüche

1. Implantat für eine Osteosynthesevorrichtung, insbesondere der Wirbelsäule, das Folgendes umfasst:
- einen Haken (1), der mit einem Befestigungskopf (2), bestehend aus zwei seitlichen Armen (3, 4) in offenem U und dazu bestimmt, einen Verbindungsschaft (5) zu seiner Immobilisierung aufzunehmen, über eine Gewindemutter (6) fest verbunden ist, die sich dazu eignet, auf die entsprechenden Gewindeteile (7) geschraubt zu werden, die auf den seitlichen Armen (3, 4) des Befestigungskopfes angeordnet sind,
- einen Gegenhaken (9), der zum Haken (1) gerichtet und frei mit dem Haken (1) verbunden ist, um es dem Haken (1) und dem Gegenhaken (9) zu erlauben, mindestens einen Wirbel (12) wie eine Zwinge unabhängig vom Verbindungsschaft (5) einzuschließen,
- und Mittel zum Blockieren des Hakens (1) in Bezug auf den Gegenhaken (9), um ihre gegenseitige Immobilisierung sicherzustellen,
**dadurch gekennzeichnet, dass** es Folgendes umfasst:
- Mittel zum freien Verbinden des Hakens (1) mit dem Gegenhaken (9), die mittels eines Zapfens (10) gebildet sind, der eines der Enden (9a) des Gegenhakens verlängert und in einer Bohrung (11) gleiten kann, die in einem oberen Teil (1a) des Hakens (1) mit dem Befestigungskopf (2) nach einer Achse Y-Y' senkrecht zur gemeinsamen Achse X-X' des Befestigungskopfes (2) und des Hakens (1) hergestellt ist,
- Mittel (13, 13A, 13B) zum Halten des Hakens (1) und des Gegenhakens (9) in relativer Stellung zueinander.

2. Implantat nach Anspruch 1, **dadurch gekennzeichnet, dass** die Mittel (13, 13A, 13B) zum Halten in Stellung aus einem elastisch verformbaren Element (15, 21, 25) bestehen, das von dem Haken getragen wird und dazu bestimmt ist, einerseits wie eine Ratsche mit Rasten (20), die auf dem Zapfen (10) angeordnet sind, beim Festziehen durch Annähern des Hakens und des Gegenhakens zusammenzuwirken und, andererseits, das Befreien des Zapfens (10) durch eine Einwirkung auf das verformbare Element zu erlauben.

3. Implantat nach Anspruch 1, **dadurch gekennzeichnet, dass** die Mittel zum Blockieren des Hakens (1) und des Gegenhakens (9) in definierter Stellung mittels des Verbindungsschafts (5), dessen Festziehen das Blockieren des Zapfens (10) sicherstellt, gebildet sind.

4. Implantat nach Anspruch 2 oder 3, **dadurch gekennzeichnet, dass** der Zapfen (10) angepasst ist, um im Inneren der Bohrung (11) zu gleiten, die mit dem Boden des von den seitlichen Armen (3, 4) gebildeten Us schneidend eingerichtet und dazu bestimmt ist, den Verbindungsschaft (5) aufzunehmen, sodass das Festziehen des Verbindungsschafts (5) durch die Mutter (6) gleichzeitig das endgültige Festziehen des Verbindungsschafts (5) und des Zapfens (10) auf dem Haken (1) sicherstellt.

5. Implantat nach Anspruch 1, **dadurch gekennzeichnet, dass** die Achse (Y-Y') der Bohrung (11) des Hakens (1), mit der der Zapfen (10) des Gegenhakens (9) zusammenarbeitet, zur gemeinsamen senkrechten Achse (X-X') des Befestigungskopfs (2) und des Hakens (1) senkrecht steht und sich in einer Ebene befindet, die mit der des Hakens (1) identisch ist, so dass der Haken (1) und der Gegenhaken (9) einander gegenüber gestellt werden.

6. Implantat nach Anspruch 1, **dadurch gekennzeichnet, dass** die Achse (Y-Y') der Bohrung (11) des Hakens (1), mit der der Zapfen (10) des Gegenhakens (9) zusammenwirkt, zur senkrechten gemeinsamen Achse (X-X') des Befestigungskopfes (2) und des Hakens (1) senkrecht steht und in einer anderen Ebene als der Symmetrieebene des Hakens (1) liegt und daher den Haken (1) und den Gegenhaken (9) winkelig zueinander und nach einer sogenannten "rechten" und einer so genannten "linken" Richtung versetzt.

7. Implantat nach einem der Ansprüche 1 bis 5, **dadurch gekennzeichnet, dass** der Gegenhaken (9) mit dem Zapfen (10) über ein Gelenk (14) verbunden ist, das ihn relativ zur Achse (Y-Y') frei drehbar macht.

8. Implantat nach einem der Ansprüche 1 bis 6, **dadurch gekennzeichnet, dass** der Befestigungskopf (2) relativ zum Haken (1) frei drehbar montiert ist.

9. Implantat nach einem der Ansprüche 2 bis 8, **dadurch gekennzeichnet, dass** das elastisch verformbare Element aus einer Nadel (15) besteht, von der ein Arm (16) in einer Aufnahme (17) befestigt ist, deren Längsachse sich im Wesentlichen senkrecht zur Ebene des Hakens (1) im oberen Teil (1a) des Hakens (1) erstreckt, und deren anderer Arm (18) elastisch verformbar ist und sich frei in eine zylindrische Bohrung (19) fügt, die zur ersten Aufnahme (17), die in den oberen Teil (1a) des Hakens (1) gebohrt ist, im Wesentlichen parallel ist, wobei sich die Bohrung (11) und die zylindrische Bohrung (19) schneiden und im Wesentlichen senkrecht zueinander stehen, damit der elastische Arm (18) wie eine Ratsche mit Rasten (20), die quer auf dem Zapfen (10) des Gegenhakens (9) angeordnet sind, beim Festziehen durch Verschieben zum Haken (1) zusammenarbeiten oder das Befreien des Zapfens (10) durch Senken des elastischen Arms (18) erlauben kann.

10. Implantat nach einem der Ansprüche 2 bis 8, **dadurch gekennzeichnet, dass** das elastisch verformbare Element aus einer Nadel (21) besteht, von der ein Arm (22) in einer Aufnahme (23) befestigt ist, deren Achse sich im Wesentlichen senkrecht zur Ebene des Hakens (1) im oberen Teil (1a) erstreckt, und deren anderer Arm (24) elastisch verformbar ist und sich frei und parallel zum ersten außerhalb des Hakens (1) erstreckt, um mit dem Zapfen (10) des Gegenhakens (9) zusammenzuarbeiten, der zu ihm im Wesentlichen senkrecht steht, damit der elastische Arm (24) wie eine Ratsche mit Rasten (20), die quer auf dem Zapfen (10) des Gegenhakens (9) angeordnet sind, beim Spannen durch Verschieben zum Haken (1) zusammenarbeiten oder das Befreien des Zapfens (10) durch Senken des elastischen Arms (24) erlauben kann.

11. Implantat nach einem der Ansprüche 2 bis 8, **dadurch gekennzeichnet, dass** das elastisch verformbare Element aus einer elastisch verformbaren Klinge (25) besteht, die parallel zur Ebene des Hakens (1), parallel zum Zapfen (10) des Gegenhakens (9) befestigt ist, und deren freies Ende (25a) zum Zapfen (10) zurückgebogen ist, damit das freie Ende (25a) wie eine Ratsche mit Rasten (20), die quer auf dem Zapfen (10) des Gegenhakens (9) eingerichtet sind, beim Festziehen durch Verschieben zum Haken (1) zusammenarbeiten oder das Freigeben des Zapfens durch Ziehen an der Klinge (25) erlauben kann.

12. Implantat nach einem der Ansprüche 1 bis 11, **dadurch gekennzeichnet, dass** der Gegenhaken (9) mit einer pedikulären Schraube ausgestattet ist.

## Claims

1. An implant for an osteosynthesis device, in particular of the spine, the implant comprising :
- a hook (1) secured to a fixing head (2) constituted by two lateral branches (3, 4) in an open U-shape designed to receive a link rod (5) for the purpose of holding it fixed relative thereto by means of a tapped nut (6) suitable for being screwed onto corresponding threaded portions (7) made on the lateral branches (3, 4) of the fixing head,
- a counterhook (9) directed towards the hook (1) and freely connected to said hook (1) to enable the hook (1) and the counterhook (9) to clamp onto at least one vertebra (12), independently of the link rod (5), and
- means for locking the hook (1) relative to the counterhook (9) so as to prevent them from moving relative to each other,
the implant being **characterised in that** it includes:
- free connection means between the hook (1) and the counterhook (9), constituted by means of a finger (10) extending one of the ends (9a) of the counterhook and adapted to slide in a bore (11) formed in a top portion (1a) of the hook (1) with the fixing head (2) along an axis Y-Y' that is perpendicular to the common axis X-X' of the fixing head (2) and of the hook (1), and
- position-maintaining means (13, 13A, 13B) for holding the hook (1) and the counterhook (9) in position relative to each other.

2. An implant according to claim 1, **characterised in that** the position-maintaining means (13, 13A, 13B) are constituted by a resiliently deformable element (15, 21, 25) borne by the hook and designed firstly to cooperate during clamping by moving the hook and the counterhook towards each other like a pawl with notches (20) formed in the finger (10), and secondly to allow the finger (10) to be released by acting on the deformable element.

3. An implant according to claim 1, **characterised in that** the means for definitively locking the hook (1) and the counterhook (9) in position are constituted by the link rod (5) which locks the finger (10) when it is itself clamped.

4. An implant according to claim 2 or 3, **characterised in that** the finger (10) is adapted to slide inside the bore (11) formed to intersect the bottom of the U-shape defined by the lateral branches (3, 4) and designed to receive the link rod (5) by the nut (6) simultaneously providing definitive clamping of the link rod (5) and of the finger (10) on the hook (1).

5. An implant according to claim 1, **characterised in that** the axis (Y-Y') of the bore (11 ) of the hook (1 ) with which the finger (10) of the counterhook (9) co-operates is perpendicular to the common vertical axis (X-X') of the fixing head (2) and of the hook (1), and is situated in a plane identical to the plane of the hook (1), thereby ensuring that the hook (1) and the counterhook (9) are put face to face.

6. An implant according to claim 1, **characterised in that** the axis (Y-Y') of the bore (11) of the hook (1) with which the finger (10) of the counterhook (9) co-operates is perpendicular to the common vertical axis (X-X') of the fixing head (2) and of the hook (1), and is situated in a plane different from the plane of symmetry of the hook (1), thereby angularly offsetting the hook (1) and the counterhook (9) relative to each other in a direction "to the right" or in a direction "to the left".

7. An implant according to one of claims 1 to 5, **characterised in that** the counterhook (9) is connected to the finger (10) by means of a hinge (14), allowing it to pivot freely about the axis (Y-Y').

8. An implant according to one of claims 1 to 6, **characterised in that** the fixing head (2) is mounted to rotate freely relative to the hook (1).

9. An implant according to one of claims 2 to 8, **characterised in that** the elastically deformable element is constituted by a pin (15) having one branch (16) fixed in a housing (17) whose longitudinal axis extends substantially perpendicularly to the plane of the hook (1) in the top portion (1a) of said hook (1), and whose other branch (18) is elastically deformable and is freely received in a cylindrical hole (19) substantially parallel to the first housing (17) bored through the top portion (1 a) of the hook (1), the bore (11) and the cylindrical hole (19) and the cylindrical hole (19) intersecting substantially perpendicularly so that when the counterhook (9) is being clamped by being moved towards the hook (1), the resilient branch (18) can co-operate like a pawl with notches (20) formed transversely on the finger (10), or can allow the finger (10) to be released by said resilient branch (18) being lowered.

10. An implant according to one of claims 2 to 8, **characterised in that** the elastically deformable element is constituted by a pin (21) having one branch (22) fixed in a housing (23) whose axis extends substantially perpendicularly to the plane of the hook (1 ) in the top portion (1 a) thereof, and whose other branch (24) is elastically deformable and extends freely and parallel to the first, outside the hook (1) so as to co-operate with the finger (10) of the counterhook (9) which is substantially perpendicular thereto so that during clamping by moving the counterhook (9) towards the hook (1) said resilient branch (24) can co-operate like a pawl with notches (20) formed transversely on the finger (10), or can allow the finger (10) to be released by said resilient branch (24) being lowered.

11. An implant according to one of claims 2 to 8, **characterised in that** the elastically deformable element is constituted by an elasticity deformable blade (25) fixed parallel to the plane of the hook (1), parallel to the finger (10) of the counterhook (9), and having a free end (25a) curved towards the finger (10) so that while the counterhook (9) is being clamped by being moved towards the hook (1), said free end (25a) can co-operate like a pawl with notches (20) formed transversely on the finger (10), or can enable said finger to be released when traction is applied to the blade (25).

12. An implant according to one of claims 1 to 11, **characterised in that** the counterhook (9) is fitted with a pedicular screw.
